# EUROPEAN PATENT SPECIFICATION

(11) **EP 1 991 671 B1**
(45) Date of publication and mention of the grant of the patent: **23.05.2012**
(21) Application number: 07713917.8
(22) Date of filing: 01.02.2007
(51) Int. Cl.: C12N 9/10, C12C 11/00, C12Q 1/68

(54) **GENE ENCODING GLYCOGEN SYNTHASE AND USE THEREOF**
FÜR GLYCOGEN-SYNTHASE CODIERENDES GEN UND VERWENDUNG DAVON
GENE CODANT POUR UNE GLYCOGENE SYNTHASE ET SON UTILISATION

(30) Priority: 28.02.2006 JP 2006054195
(43) Date of publication of application: 19.11.2008
(73) Proprietor: Suntory Holdings Limited, Kita-ku, Osaka-shi, Osaka 530-8203 (JP)
(72) Inventor: NAKAO, Yoshihiro, Mishima-gun, Osaka 618-8503 (JP); KODAMA, Yukiko, Mishima-gun, Osaka 618-8503 (JP); SHIMONAGA, Tomoko, Mishima-gun, Osaka 618-8503 (JP)
(74) Representative: Vossius & Partner
(86) International application number: PCT/JP2007/052170
(87) International publication number: WO 2007/099751

(56) References cited:
- FARKAS I ET AL: "ISOLATION OF THE GSY1 GENE ENCODING YEAST GLYCOGEN SYNTHASE AND EVIDENCE FOR THE EXISTENCE OF A SECOND GENE" JOURNAL OF BIOLOGICAL CHEMISTRY, vol. 265, no. 34, 1990, pages 20879-20886, XP002428006 ISSN: 0021-9258
- SEO BEOM-SEOK ET AL: "Functional interactions between heterologously expressed starch-branching enzymes of maize and the glycogen synthases of Brewer's yeast" PLANT PHYSIOLOGY (ROCKVILLE), vol. 128, no. 4, April 2002 (2002-04), pages 1189-1199, XP002428007 ISSN: 0032-0889

## Description

### TECHNICAL FIELD

The present invention relates to a yeast as defined in the claims with superior resistance property to dryness, and a method for producing alcoholic beverages by using the yeast of the invention.

More particularly, the present invention relates to a yeast, whose resistance property to dryness is enhanced by amplifying expression level of non-ScGSY1 gene specific to a lager brewing yeast and to a method for producing alcoholic beverages with said yeast, etc. Further, the yeast of the present invention is useful as a baker's yeast or an industrial yeast as well.

### BACKGROUND ART

Beer brewing is characterized by a process recovering yeasts after fermentation and using the recovered yeasts at the subsequent fermentation, which is called "Renjo". The yeasts are stored in the presence of ethanol in a tank whose temperature is kept at approximately 0 to 3°C. When the yeasts die during the storage, not only the next fermentation process is interfered, but also constituents of the yeast cells released by cell lysis may impart unfavorable taste to product Therefore, it is very important for allowing some variance to design production process and for stable production of quality products to use yeasts with superior resistant property to low temperature storage.

"Renjo" may be terminated at a certain times of fermentation is carried out. The number of times of "Renjo" may vary according to fermentation conditions or properties of yeasts used in the, process. A process to develop yeasts for fermentation freshly is called propagation. Yeasts are subcultured several times enlarging scales of culture successively during the propagation process. Because propagation process requires from several days to several weeks, it brings great advantages in production efficiency if term of the process is shortened or yeast cells which are large-scale pre-cultured are able to be stored stably for extended period of time at low temperature or under dry condition.

Concerning a method for producing dry yeast maintaining high viable cell ratio, improvement of drying equipment, or improvement of manufacturing conditions such as temperature or addition of emulsifiers, etc. have been made. For example, L-drying method is not practical to be used at industrial production scale because, though it can maintain extremely high viable cell ratio, but at the same time it takes a lot of time and cost.

Regarding low-temperature resistance of yeast, some experiments designed to improve refrigeration-resistant property mainly of baker's yeast were reported. This is because Saccharomyces cerevisiae, which is a baker's yeast, has poor low-temperature storage property in comparison with brewer's yeast for beer or sake, which can ferment at low temperature. For example, baker's yeasts having refrigeration-resistarn property and drying-resistant property were found out mainly by screening methods in Japanese Patent Application Laid-open No. H11-155559 and Japanese Patent Application Laid-open No. 2003-304864. Further, regarding examples utilizing genetic engineering techniques, trehalose highly accumulating strains by disruption of NTH1, which is a trehalase gene, is reported in Japanese Patent Application Laid-open No. H10-117771 and a strain highly accumulating specific amino acids such as arginine by disruption of CAR1, which is an arginase gene, is reported in Japanese Patent Application Laid-open No. 2001-238665.

### DISCLOSURE OF INVENTION

Under the above situations, there has been a need to make high-efficiency production of alcoholic beverages or useful materials possible by using a gene encoding a protein responsible for drying and/or low temperature storage-resistant property of brewery yeast and said protein.

The present inventors made extensive studies to solve the above problems and as a result, succeeded in identifying and isolating a gene encoding glycogen synthase from beer yeast. Moreover, the present inventors produced transformed yeast in which the obtained gene was expressed to verify that drying-resistant property and/or low-temperature storage-resistant property can be actually improved, thereby completing the present invention.

Thus, disclosed is a gene encoding a glycogen synthase of brewery yeast, to a protein encoded by said gene, to a transformed yeast in which the expression of said gene is controlled, to a method for enhancing drying-resistant property and/or low-temperature storage-resistant property of yeast using a yeast in which the expression of said gene is controlled, or the like. More specifically, disclosed are the following polynucleotides, a vector comprising said polynucleotide.

The present invention provides a yeast comprising a vector containing the polynucleotide as defined in the claims, a method for producing alcoholic beverages by using said yeast, and the like.

The present invention relates to:
(1) A yeast comprising a vector containing the polynucleotide selected from:
   (a) a polynucleotide comprising a polynucleotide consisting of the nucleotide sequence of SEQ ID NO: 1;
   (b) a polynucleotide comprising a polynucleotide encoding a protein consisting of the amino acid sequence of SEQ ID NO: 2;
   (c) a polynucleotide comprising a polynucleotide encoding a protein consisting of the amino acid sequence of SEQ ID NO: 2 in which 1 to 30 amino acids thereof are deleted, substituted, inserted and/or added, wherein said protein has a glycogen synthase activity and wherein amplifying expression of said protein in the yeast AJL4004 strain increases the drying-resistant property as compared to the AJL4004 strain;
   (d) a polynucleotide comprising a polynucleotide encoding a protein having an amino acid sequence having 95% or higher identity with the amino add sequence of SEQ ID NO: 2, wherein said protein has a glycogen synthase activity and wherein amplifying expression of said protein in the yeast AJL4004 strain increases the drying-resistant property as compared to the AJL4004 strain;
   (e) a polynucleotide comprising a polynucleotide encoding a protein consisting of the amino acid sequence of SEQ ID NO: 2, or encoding the amino acid sequence of SEQ ID NO: 2 in which 1 to 7 amino acids thereof are deleted, substituted, inserted, and/or added, and wherein said protein has a glycogen synthase activity and wherein amplifying expression of said protein in the yeast AJL4004 strain increases the drying-resistant property as compared to the AJL4004 strain; and
   (f) a polynucleotide comprising a polynucleotide encoding a protein having 99% or higher identity with the amino acid sequence of SEQ ID NO: 2, and having a glycogen synthase activity and wherein amplifying expression of said protein in the yeast AJL4004 strain increases the drying-resistant property as compared to the AJL4004 strain,
   wherein drying-resistant property is increased by increasing an expression level of the protein encoded by the polynucleotide according to any one of (a) to (f).
(2) A method for producing an alcoholic beverage by using the yeast according to (1).
(3) A method for producing an alcoholic beverage comprising: conducting fermentation using the yeast according to (1).

Also disclosed is:
(1) A polynucleotide selected from the group consisting of
   (a) a polynucleotide comprising a polynucleotide consisting of the nucleotide sequence of SEQ ID NO: 1 or SEQ ID NO: 3;
   (b) a polynucleotide comprising a polynucleotide encoding a protein consisting of the amino acid sequence of SEQ ID NO: 2 or SEQ ID NO: 4;
   (c) a polynucleotide comprising a polynucleotide encoding a protein consisting of the amino acid sequence of SEQ ID NO: 2 or SEQ ID NO: 4 in which one or more amino acids thereof are deleted, substituted, inserted and/or added, and having a glycogen synthase activity;
   (d) a polynucleotide comprising a polynucleotide encoding a protein having an amino acid sequence having 60% or higher identity with the amino acid sequence of SEQ ID NO: 2 or SEQ ID NO: 4, and said protein having a glycogen synthase activity;
   (e) a polynucleotide comprising a polynucleotide which hybridizes to a polynucleotide consisting of a nucleotide sequence complementary to the nucleotide sequence of SEQ ID NO: 1 or SEQ ID NO: 3 under stringent conditions, and which encodes a protein having a glycogen synthase activity, and
   (f) a polynucleotide comprising a polynucleotide which hybridizes to a polynucleotide consisting of a nucleotide sequence complementary to the nucleotide sequence of the polynucleotide encoding the protein having the amino acid sequence of SEQ ID NO: 2 or SEQ ID NO: 4 under stringent conditions, and which encodes a protein having a glycogen synthase activity.
(2) The polynucleotide according to (1) above selected from the group consisting of:
   (g) a polynucleotide comprising a polynucleotide encoding a protein consisting of the amino acid sequence of SEQ ID NO: 2 or SEQ ID NO: 4, or encoding the amino acid sequence of SEQ ID NO: 2 or SEQ ID NO: 4 in which 1 to 10 amino adds thereof are deleted, substituted, inserted, and/or added, and wherein said protein has a glycogen synthase activity;
   (h) a polynucleotide comprising a polynucleotide encoding a protein having 90% or higher identity with the amino acid sequence of SEQ ID NO: 2 or SEQ ID NO: 4, and having a glycogen synthase activity; and
   (i) a polynucleotide comprising a polynucleotide which hybridizes to a polynucleotide consisting of a nucleotide sequence of SEQ ID NO: 1 or SEQ ID NO: 3 or which hybridizes to a polynucleotide consisting of a nucleotide sequence complementary to the nucleotide sequence of SEQ ID NO: 1 or SEQ ID NO: 3, under high stringent conditions, which encodes a protein having a glycogen synthase activity.
(3) The polynucleotide according to (1) above comprising a polynucleotide consisting of the nucleotide sequence of SEQ ID NO: 1 or SEQ ID NO: 3.
(4) The polynucleotide according to (1) above comprising a polynucleotide encoding a protein consisting of the amino acid sequence of SEQ ID NO: 2 or SEQ ID NO: 4.
(5) The polynucleotide according to any one of (1) to (4) above, wherein the polynucleotide is DNA
(6) A protein encoded by the polynucleotide according to any one of (1) to (5) above.
(7) A vector containing the polynucleotide according to any one of (1) to (5) above.
(7a) The vector of (7) above, which comprises the expression cassette comprising the following components:
   (x) a promoter that can be transcribed in a yeast cell;
   (y) any of the polynucleotides described in (1) to (5) above linked to the promoter in a sense or antisense direction; and
   (z) a signal that can function in a yeast with respect to transcription termination and polyadenylation of a RNA molecule.
(7b) The vector of (7) above, which comprises the expression cassette comprising the following components:
   (x) a promoter that can be transcribed in a yeast cell;
   (y) any of the polynucleotides described in (1) to (5) above linked to the promoter in a sense direction; and
   (z) a signal that can function in a yeast with respect to transcription termination and polyadenylation of a RNA molecule.
(8) A yeast into which the vector according to any one of (7) to (7b) above has been introduced.
(9) The yeast (yeast for practical use) according to (8) above, wherein drying-resistant property is increased. The "yeast for practical use" means that a yeast which possesses practical value such as brewer's (brewery) yeast, baker's yeast or industrial yeast, etc.
(10) The yeast according to (8) above, wherein low-temperature storage-resistant property is increased.
(11) The yeast according to (9) above, wherein the drying-resistant property is increased by increasing an expression level of the protein of (6) above.
(12) The yeast according to (10) above, wherein the low-temperature storage-resistant property is increased by increasing an expression level of the protein of (6) above.
(12a) The yeast according to any one of (9) to (12) above, wherein the yeast is a brewery yeast.
(13) A method for producing an alcoholic beverage by using the yeast according to any one of (8) to (12a) above.
(14) The method according to (13) above, wherein the brewed alcoholic beverage is a malt beverage.
(15) The method according to (13) above, wherein the brewed alcoholic beverage is wine.
(16) An alcoholic beverage produced by the method according to any one of (13) to (15) above.
(17) A method for assessing a test yeast for its drying-resistant property and/or low-temperature storage-resistant property, comprising using a primer or probe designed based on the nucleotide sequence of a gene having the nucleotide sequence of SEQ ID NO: 1 or SEQ ID NO: 3 and encoding a protein having a glycogen synthase activity.
(17a) A method for selecting a yeast having an increased drying-resistant property and/or low-temperature storage-resistant property by using the method described in (17) above.
(17b) A method for producing an alcoholic beverage (for example, beer or alcohol for industrial use, etc.) by using the yeast selected with the method described in (17a) above.
(17c) A method for producing an useful materials (for example, protein) by using the yeast selected with the method described in (17a) above.
(18) A method for assessing a test yeast for its drying-resistant property and/or low-temperature storage-resistant property, comprising: culturing the test yeast; and measuring the expression level of the gene having the nucleotide sequence of SEQ ID NO: 1 or SEQ ID NO: 3 and encoding a protein having a glycogen synthase activity.
(18a) A method for selecting a yeast having a high drying-resistant property and/or low-temperature storage-resistant property, which comprises assessing a test yeast by the method described in (18) above and selecting a yeast having a high expression level of gene encoding a protein having a glycogen synthase activity.
(18b) A method for producing an alcoholic beverage (for example, beer) by using the yeast selected with the method described in (18a) above.
(18c) A method for producing an useful material (for example, protein) by using the yeast selected with the method described in (18a) above.
(19) A method for selecting a yeast, comprising: culturing test yeasts; quantifying the protein of (6) above or measuring the expression level of the gene having the nucleotide sequence of SEQ ID NO: 1 or SEQ ID NO: 3 and encoding a protein having a glycogen synthase activity; and selecting a test yeast having an amount of the protein or the gene expression level according to favorable drying-resistant property and/or low-temperature storage-resistant property.
(20) The method for selecting a yeast according to (19) above, comprising: culturing a reference yeast and test yeasts; measuring for each yeast the expression level of the gene having the nucleotide sequence of SEQ ID NO: 1 or SEQ ID NO: 3 and encoding a protein having a glycogen synthase activity, and selecting a test yeast having the gene expression higher than that in the reference yeast.
(21) The method for selecting a yeast according to (19) above, comprising: culturing a reference yeast and test yeasts; quantifying the protein according to (6) above in each yeast; and selecting a test yeast having a larger amount of the protein than that in the reference yeast.
(22) A method for producing an alcoholic beverage comprising: conducting fermentation using the yeast according to any one of (8) to (12a) above or a yeast selected by the methods according to any one of (19) to (21) above.

The transformed yeast of the present invention is able to keep high viable cell ratio during dry storage. Therefore, when it is used for brewing and so on, painfulness of conserving yeast can be eliminated. Further, it is expected to contribute to quality stabilization. Moreover, dry yeast is suitable for long-storage, and it is very advantageous to distribution or transportation due to its reduced weight It is also useful as microorganisms for industrial application such as industrial alcohol production or production of useful proteins. The yeast of the present invention also useful as an industrial yeast as well.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test. The horizontal axis represents fermentation time while the vertical axis represents apparent extract concentration (w/w%).
Figure 3 shows the expression profile of non-ScGSY1 gene in yeasts upon beer fermentation test. The horizontal axis represents fermentation time while the vertical axis represents the intensity of detected signal..
Figure 4 shows the result of drying-resistant property test of parent strain and non-ScGSY1 highly expressed strain.
Figure 5 shows the cell growth with time upon beer fermentation test. The horizontal axis represents fermentation time while the vertical axis represents optical density at 660 nm (OD660).

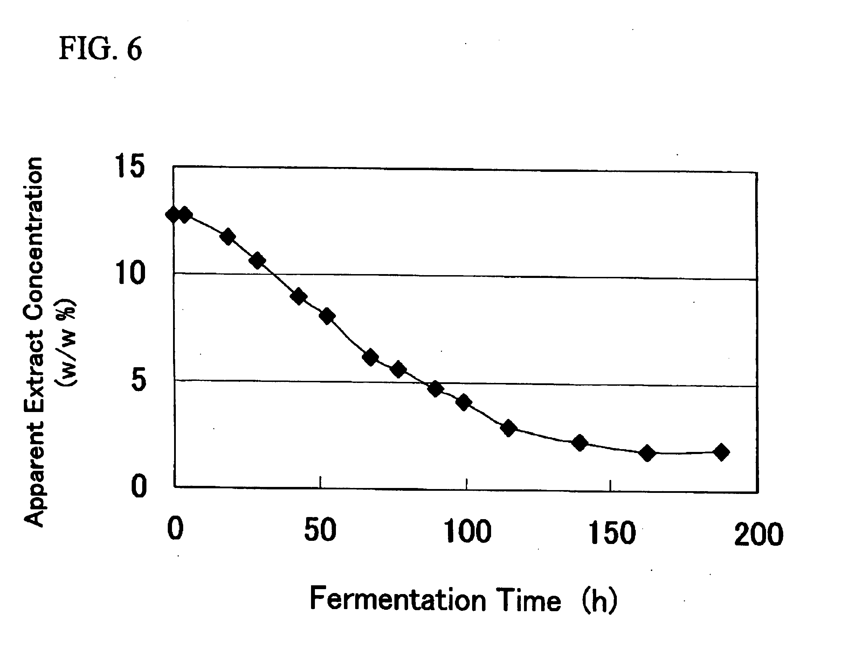

Figure 6 shows the extract (sugar) consumption with time upon beer fermentation test. The horizontal axis represents fermentation time while the vertical axis represents apparent extract concentration (w/w%).
Figure 7 shows the expression profile of non-ScGSY2 gene in yeasts upon beer fermentation test. The horizontal axis represents fermentation time while the vertical axis represents the intensity of detected signal.

### BEST MODES FOR CARRYING OUT THE INVENTION

The present inventors isolated and identified non-ScGSY1 gene and non-ScGSY2 gene encoding glycogen synthases of brewery yeast based on the lager brewing yeast genome information mapped according to the method disclosed in Japanese Patent Application Laid-Open No. 2004-283169. The nucleotide sequences of the genes are represented by SEQ ID NO: 1 and SEQ ID NO: 3. Further, amino acid sequences of proteins encoded by the genes are represented by SEQ ID NO: 2 and SEQ ID NO: 4, respectively.

### 1. Polynucleotide

First of all, disclosed: . (a) a polynucleotide comprising a polynucleotides of the nucleotide sequence of SEQ ID NO: 1 or SEQ ID NO: 3; and (b) a polynucleotide comprising a polynucleotide encoding a protein of the amino acid sequence of SEQ ID NO: 2 or SEQ ID NO: 4. The polynucleotide can be DNA or RNA.

The target polynucleotide is not limited to the polynucleotide encoding a protein having a glycogen synthase activity described above and may include other polynucleotides encoding proteins having equivalent functions to said protein. Proteins with equivalent functions include, for example, (c) a protein of an amino acid sequence of SEQ ID NO: 2 or SEQ ID NO: 4 with one or more amino acids thereof being deleted, substituted, inserted and/or added and having a glycogen synthase activity.

Such proteins include a protein consisting of an amino acid sequence of SEQ ID NO: 2 or SEQ ID NO: 4 with, for example, 1 to 100, 1 to 90, 1 to 80, 1 to 70, 1 to 60, 1 to 50, 1 to 40, 1 to 39, 1 to 38, 1 to 37, 1 to 36, 1 to 35, 1 to 34, 1 to 33, 1 to 32,1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted; substituted, inserted and/or added and having a glycogen synthase activity. In general, the number of deletions, substitutions, insertions, and/or additions is preferably smaller. In addition, such proteins include (d) a protein having an amino acid sequence with about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2 or SEQ ID NO: 4, and having a glycogen synthase activity. In general, the percentage identity is preferably higher.

Glycogen synthase activity may be measured, for example, by a method described in Cheng et al., Mol. Cell. Biol., 15(12), 6632-6640 (1995).

Furthermore, also disclosed is (e) a polynucleotide comprising a polynucleotide which hybridizes to a polynucleotide consisting of a nucleotide sequence complementary to the nucleotide sequence of SEQ ID NO: 1 or SEQ ID NO: 3 under stringent conditions and which encodes a glycogen synthase; and (f) a polynucleotide comprising a polynucleotide which hybridizes to a polynucleotide complementary to a nucleotide sequence of encoding a protein of SEQ ID NO: 2 or SEQ ID NO: 4 under stringent conditions, and which encodes a glycogen synthase.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or SEQ ID NO: 3 or polynucleotide encoding the ammo acid sequence of SEQ ID NO: 2 or SEQ ID NO: 4 as a probe. The hybridization method may be a method described, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher; 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO: 2 or SEQ ID NO: 4 as calculated by homology search software, such as FASTA and BLAST using default parameters.

Identity between amino acid sequences or nucleotide sequences may be determined using algorithm BLAST by Karlin and Altschul (Proc. Natl Acad Sci. USA, 87: 2264-2268, 1990; Proc. Natl. Acad. Sci. USA, 90:5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score =100 and word length = 12. When an amino acid sequence is sequenced using BLASTX, the parameters are, for example, score = 50 and word length = 3. When BLAST and Gapped BLAST programs are used, default parameters for each of the programs are employed.

### 2. Protein

Also disclosed are proteins encoded by any of the polynucleotides (a) to (i) above. A preferred protein comprises an amino acid sequence of SEQ ID NO: 2 or SEQ ID NO: 4 with one or several amino acids thereof being deleted, substituted, inserted and/or added, and having a glycogen synthase activity.

Such protein includes those having an amino acid sequence of SEQ ID NO: 2 or SEQ ID NO: 4 with amino acid residues thereof of the number mentioned above being deleted, substituted, inserted and/or added and having a glycogen synthase activity. In addition, such protein includes those having homology as described above with the amino acid sequence of SEQ ID NO: 2 or SEQ ID NO: 4 and having a glycogen synthase activity.

Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad. Sci. USA 79: 6409 (1982), Gene 34: 315 (1985), Nuc. Acids. Res., 13: 4431 (1985), Proc. Natl. Acad. Sci. USA 82: 488 (1985).

Deletion, substitution, insertion and/or addition of one or more amino acid residues in an amino acid sequence of the protein means that one or more amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PekinElmer, Pharmacia, Protein Technology Instrument, Synthecell-Vega, PerSeptive, Shimazu Corp. can also be used for chemical synthesis.

### 3. Vector and yeast transformed with the vector

Disclosed is a vector comprising the polynucleotide described above. The vector is directed to a vector including any of the polynucleotides described in (a) to (i) above or any of the polynucleotides described in (j) to (m) above. Generally, the vector comprises an expression cassette including as components (x) a promoter that can transcribe in a yeast cell; (y) a polynucleotide described in any of (a) to (i) above that is linked to the promoter in sense or antisense direction; and (z) a signal that functions in the yeast with respect to transcription termination and polyadenylation of RNA molecule. Further, in order to highly express the protein disclosed herein, these polynucleotides are preferably introduced in the sense direction to the promoter to promote expression of the polynucleotide (DNA) described in any of (a) to (i) above.

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad Sci. USA, 76: 1035, 1979) is known as a YIp type vector, all of which are readily available.

Promoters/terminators for adjusting gene expression in yeast may be in any combination as long as they function in the yeast for practical use and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a yeast for practical use, for example, a geneticin-resistant gene (G418r), a copper-resistant gene (CUP1) (main et al., Proc. Natl. Acad. Sci. USA, 81, 337 1984) or a cerulenin-resistant gene (fas2m, PDR4) (Junji Inokoshi et al., Biochemistry, 64, 664, 1992; and Hussain et al., Gene, 101: 149, 1991, respectively) may be used.

A vector constructed as described above is introduced into a host yeast. Examples of the host yeast include any yeast (yeast for practical use) that can be used for brewing, for example, brewery yeasts for beer, wine and sake, baker's yeast, yeast for producing industrial alcohol or yeast for producing useful proteins and so on. Specifically, yeasts such as genus *Saccharomyces* may be used. According to the present invention, a lager brewing yeast, for example, *Saccharomyces pastorianus* W34/10, etc., *Saccharomyces carlsbergenis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan, baker's yeast such as NBRC0555, NBRC1346 or NBRC2043, etc., may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl. Acad. Sci. USA, 75: 1929(1978)), a lithium acetate method (J. Bacteriology, 153: 163 (1983)), and methods described in Proc. Natl. Acad. Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering Vol. 1, Plenum Press, New York, 117(1979)), etc.) such that OD600 mn will be 1 to 6. This culture yeast is collected by centrifugation, washed and pre-treated with alkali metal ion, preferably lithium ion at a concentration of about 1 to 2 M After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes, Thereafter, it is seeded to a standard agar medium containing an antibiotic or the like as a selective marker to obtain a transformant.

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

A yeast having a superior drying-resistant property can be obtained by introducing the vector as defined in the claims to a yeast. Further, a yeast having a superior drying-resistant property can be obtained by selecting a yeast by the yeast assessment method described below. The target use of yeasts of the present invention include, for example, but not limited to, brewing alcoholic beverages such as beer, wine, whisky, sake and the like, baking bread, manufacturing useful materials such as industrial alcohol production and production of useful proteins.

In order to produce these products, a known technique can be used except that a yeast of the present invention is used in the place of a parent strain. Since starting materials, manufacturing equipment, manufacturing control and the like may be the same as the conventional ones; it can be performed without increasing cost.

### 5. Yeast assessment method

Also disclosed is a method for assessing a test yeast for its drying-resistant property and/or low temperature storage-resistant property by using a primer or a probe designed based on a nucleotide sequence of a gene having the nucleotide sequence of SEQ ID NO: 1 or SEQ ID NO: 3 and encoding a protein having a glycogen synthase activity. General technique for such assessment method is know and is described in, for example, WO01/040514, Japanese Laid-Open Patent Application No. H8-205900 or the like. This assessment method is described in below.

First, genome of a test yeast is prepared. For this preparation, any know method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the gene encoding a protein having a slycoge synthesis activity, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the drying-resistant property and/or low-temperature storage-resistant property of yeast as determined by whether the molecular weight of the amplified product is a size that contains the DNA molecule of the specific part In addition, by analyzing the nucleotide sequence of the amplified product, the property may be predicted and/or assessed more precisely.

Moreover, a test yeast is cultured to measure an expression level of the gene encoding a protein having a glycogen synthase activity having the nucleotide sequence of SEQ ID NO: 1 or SEQ ID NO: 3 to assess the test yeast for its drying-resistant property and/or low temperature storage-resistant property. Measurement of expression level of the gene encoding a protein having a glycogen synthase activity can be performed by culturing test yeast and then quantifying mRNA or a protein resulting from the gene. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the gene encoding a protein having a glycogen synthase activity having the nucleotide sequence of SEQ ID NO: 1 or SEQ ID NO: 3 are measured to select a test yeast with the gene expression level according to the target glycogen producing ability, thereby a yeast favorable for brewing desired alcoholic beverages can be selected. In addition, a reference yeast and a test yeast may be cultured so as to measure and compare the expression level of the gene in each of the yeasts, thereby a favorable test yeast can be selected. More specifically, for example, a reference yeast and one or more test yeasts are cultured and an expression level of the gene encoding a protein having a glycogen synthase activity having the nucleotide sequence of SEQ ID NO: 1 or SEQ ID NO: 3 is measured in each yeast. By selecting a test yeast with the gene expressed higher than that in the reference yeast, a yeast suitable for brewing desired alcoholic beverages or production of useful materials can be selected.

Alternatively, test yeasts are cultured and a yeast with a high glycogen synthase activity is selected, thereby a yeast suitable for brewing desired alcoholic beverages or production of useful materials can be selected.

In these cases, the test yeasts or the reference yeast may be, for example, a yeast introduced with the vector, an artificially mutated yeast or a naturally mutated yeast. The glycogen synthase activity can be measured by, for example, a method described in Cheng et al., Mol. Cell. Biol., 15(12), 6632-6640 (1995). The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see, e.g.,* Yasuji Oshima Ed., BIOCHEMISTYRY EXPERIMENTS vol. 39, Yeast Molecu/ar Genetic Experiments, pp. 67-75, JSSP).

In addition, examples of yeasts used as the reference yeast or the test yeasts include any yeasts (yeasts for practical use), for example, brewery yeasts for beer, wine, sake and the like or baker's yeast, yeast for producing industrial alcohol or yeast for producing useful proteins, etc. More specifically, yeasts such as genus *Saccharomyces* may be used *(e.g., S. pastorianus, S. cerevisiae,* and *S. carlsbergensis).* According to the present invention, a lager brewing yeast, for example, *Saccharomyces pastorianus* W34/70; *Saccharomyces carlsbergensis* NCYC453 or *NCYC456; or Saccharomyces cerevisiae NBRC1951, NBRC1952, NBRC1953* or NBRC1954, etc., may be used. Further, wine yeasts such as wine yeasts #1, 3 and 4 from the Brewing Society of Japan; and sake yeasts such as sake yeast #7 and 9 from the Brewing Society of Japan, baker's yeast such as NBRC0555, :NBRC1346 and NBRC2043, etc., may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of Gene Encoding Glycogen synthase (non-ScGSY1)

A gene encoding a glycogen synthase of lager brewing yeast (non-ScGSY1) (SEQ ID NO: 1) was found as a result of a search utilizing the comparison database described in Japanese Patent Application Laid-Open No. 2004-283169. Based on the acquired nucleotide sequence information, primers non-ScGSY1_for (SEQ ID NO: 5) and non-ScGSY1-rv (SEQ ID NO: 6) were designed to amplify the full-length of the gene. PCR was carried out using chromosomal DNA of a genome sequencing strain, Saccharomyces pastorianus Weihenstephan 34/70 (sometimes abbreviated as "W34/70 strain"), as a template to obtain DNA fragments including the full-length gene of non-ScGSY1.

The non-ScGSY1 gene fragments thus obtained were inserted into pCR2.1-TOPO vector (Invitrogen) by TA cloning. The nucleotide sequences of the non-ScGSY1 gene were analyzed by Sanger's method (F. Sanger, Science, 214: 1215, 1981) to confirm the nucleotide sequence.

### Example 2: Analysis of Expression of non-ScGSY1 Gene during Beer Fermentation

A beer fermentation test was conducted using a lager brewing yeast, Saccharomyces pastorianus W34/70, and mRNA extracted from the lager brewing yeast during fermentation was detected by a beer yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8 × 10⁶ cells/mL |

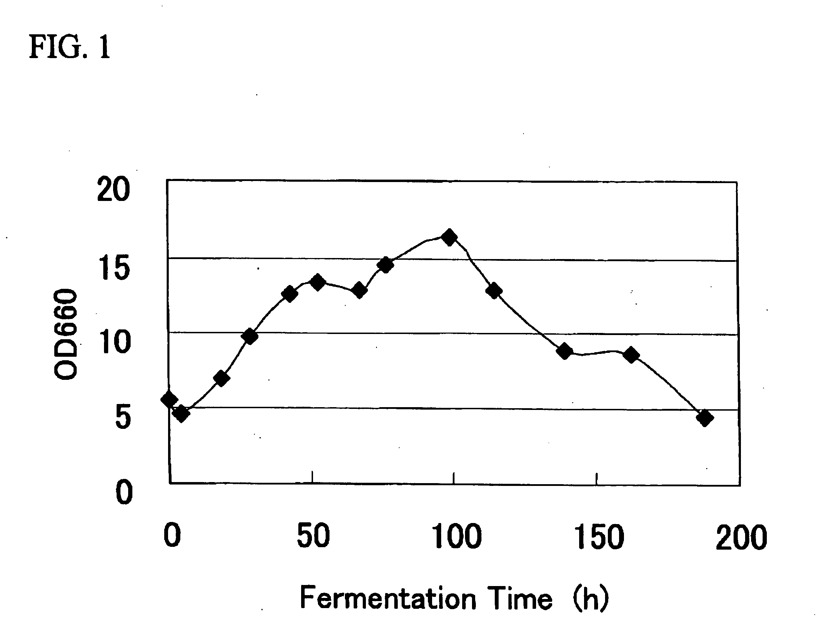

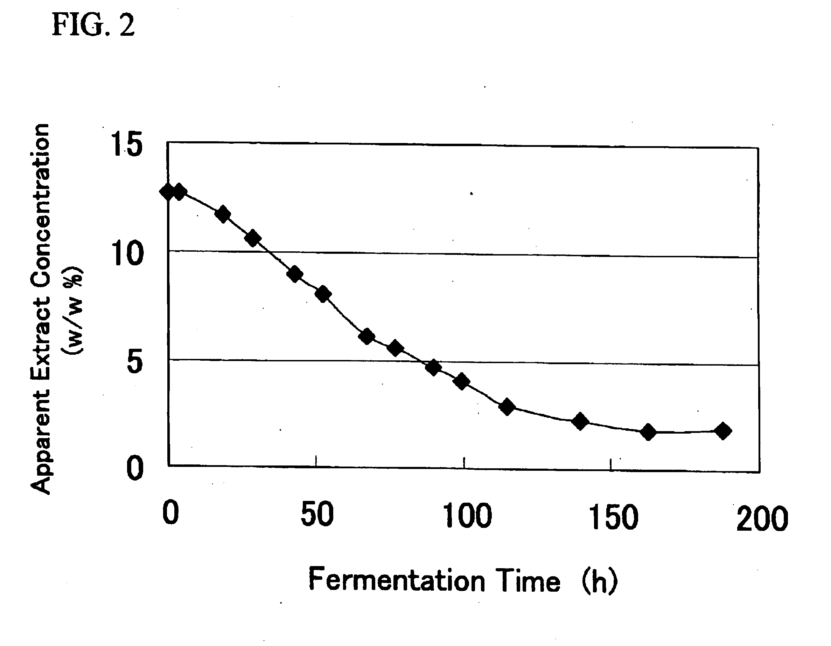

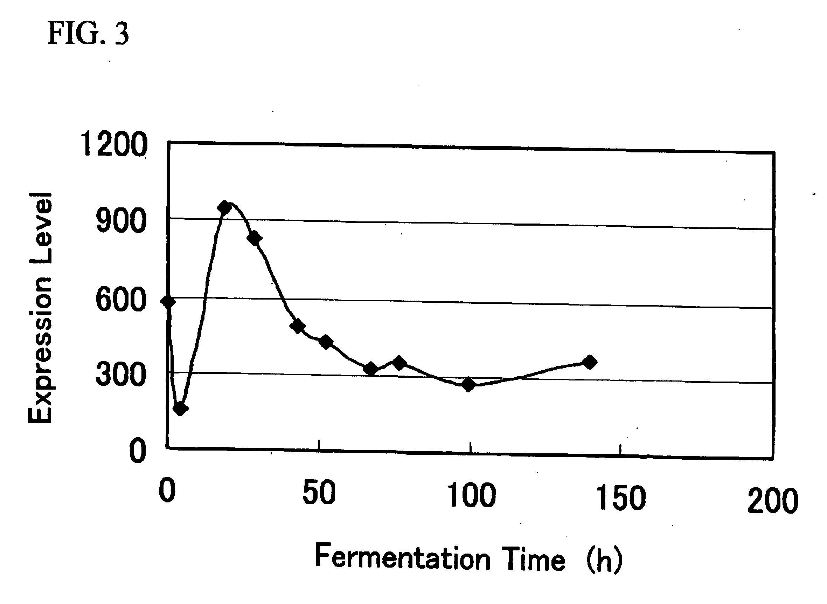

The fermentation liquor was sampled over time, and the time-course changes in amount of yeast cell growth (Fig. 1) and apparent extract concentration (Fig. 2) were observes. Simultaneously, yeast cells were sampled to prepare mRNA, and the prepared mRNA was labeled with biotin and was hybridized to a beer yeast DNA microarray. The signal was detected using GeneChip Operating system (GCOS; GeneChip Operating Software 1.0, manufactured by Affymetrix Co). Expression pattern of the non-ScGSY1 gene is shown in Figure 3. This result confirmed the expression of the non-ScGSY1 gene in the general beer fermentation

### Example 3: Construction of non-ScGSY1 Highly Expressed Strain

The non-ScGSY1pCR2.1-TOPO described in Example 1 was digested with the restriction enzymes SacI and NotI to prepare a DNA fragment containing the entire length of the protein-encoding region. This fragment was ligated to pYCGPYNot treated with the restriction enzymes SacI and NotI, thereby constructing the non-ScGSY1 high expression vector non-ScGSY1/pYCGPYNot. pYCGPYNot is a YCp-type yeast expression vector. A gene inserted is highly expressed by the pyruvate kinase gene PYK1 promoter. The geneticin-resistant gene G418^{r} is included as the selectable marker in the yeast, and the ampicillin-resistant gene Amp^{r} as the selectable marker in Escherichia coli.

Using the high expression vector prepared by the above method, an AJL4004 strain was transformed by the method described in Japanese Patent Application Laid-open No. H07-303475. The transformants were selected on a YPD plate medium (1% yeast extract, 2% polypeptone, 2% glucose and 2% agar) containing 300 mg/L of geneticin.

### Example 4: Evaluation of Drying-resistant Property of non-ScGSY1 Highly Expressed Strain

Drying-resistant properties of the parent strain (AJL4004 strain) and the non-ScGSY1 highly expressed strain obtained by the method described in Example 3 were evaluated by a method described below.

One platinum loopful of each yeast was inoculated into 10 mL of wort containing 100 mg/L of geneticin, and stirred at 30°C overnight (precultivation). The precultivation liquid was inoculated into 10 mL wort containing 100 mg/L of geneticin to make its OD660 = 0.5, then main culture was initiated. The culture was continued for 2 days until the growth of the yeast reached stationary phase. Turbidity of the culture was measured at the completion of the culture, then the culture liquid was suspended in sterile water to make its OD = 2. One hundred microliter (100 µL) of the suspension thus obtained was dispensed into a 1.5 mL microtube, then the yeast cells were dried by evaporation for 1 hour using a reduced-pressure concentrator (DNA110 SpeedVac (registered trademark), manufactured by ThermoSavant).

Viable cell ratio was measured by a method described below. The dried yeast cells obtained above were resuspended in 50 µL of sterile water, then 50 µL of 0.02% methylene blue solution (pH 4.5) was added to the suspension. Blue-stained yeast cells which had lost reducing power were considered as dead yeast cells. Then the suspension was observed under a microscope, and viable cell ratio was measured using a Cell Vital Analyzer System (DA cell counter, manufactured by Yamato Scientific Co., Ltd). The cells were counted until the population reached more than 2000 cells to minimize experimental error.

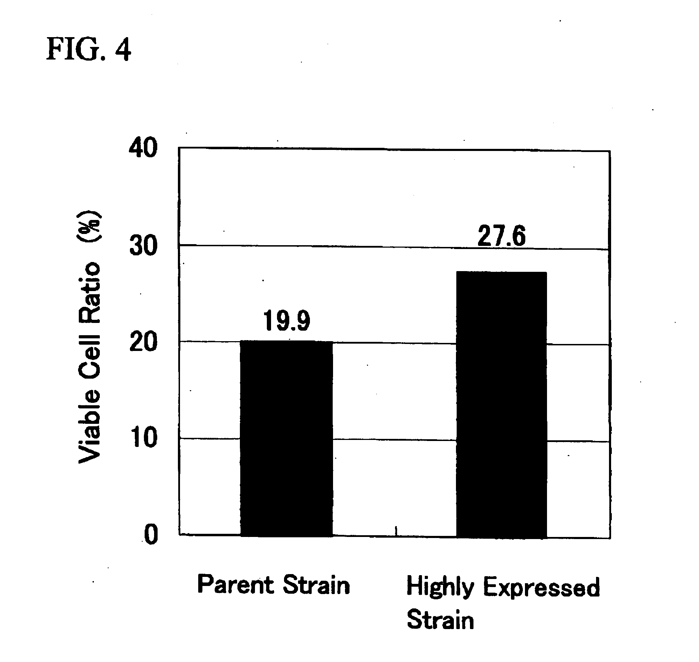

As indicated in Figure 4, viable cell ratio of the highly-expressed strain was 27.6%, though viable cell ratio of the parent strain was 19.9%. It was demonstrated by the results that drying-resistant property of yeast was increased by high expression of non-ScGSY1.

### Example 5: Evaluation of Low-temperature Resistant Property of non-ScGSY1 Highly Expressed Strain

Low-temperature resistant property of the parent strain (AJL4004 strain) and the non-ScGSY1 highly expressed strain obtained by the method described in Example 3 are evaluated by the mehtod described below. Nine hundred microliter (900 µL) of the yeast suspensions cultured by the method described in Example 4 and prepared as OD660=2 are dispensed into two microtubes, respectively. One hundred microliter (100 µL) of sterile water is added to one of the microtubes, on the other hand, 100 µL of 99.5 % ethanol is added to another one (final concentration is 10%). The suspensions are stored at 5°C for 4 weeks, then viable cell ratios are measured by the same method as Example 4.

### Example 6: Cloning of Gene Encoding Glycogen synthase (non-ScGSY2)

A gene encoding a glycogen synthase of lager brewing yeast (non-ScGSY2) (SEQ ID NO: 3) was found as a result of a search utilizing the comparison database described in Japanese Patent Application Laid-Open No. 2004-283169. Based on the acquired nucleotide sequence information, primers non-SoGSY2_for (SEQ ID NO: 7) and non-ScGSY2_rv (SEQ ID NO: 8) were designed to amplify the full-length of the gene. PCR was carried out using chromosomal DNA of a genome sequencing strain, Saccharomyces pastorianus Weihenstephan 34/70, as a template to obtain DNA fragments including the full-tength gene of on-ScGSY2.

The non-ScGSY2 gene fragments thus obtained were inserted into pCR2.1-TOPO vector (Invitrogen) by TA cloning. The nucleotide sequences of the non-ScGSY2 gene were analyzed by Sanger's method (F. Sanger, Science, 214: 1215, 1981) to confirm the nucleotide sequence.

### Example 7: Analysis of Expression of non-SeGSY2 Gene during Beer Fermentation

A beer fermentation test was conducted using a lager brewing yeast, Saccharomyces pastorianus W34/70, and mRNA extracted from the lager brewing yeast during fermentation was detected by a beer yeast DNA microarray

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

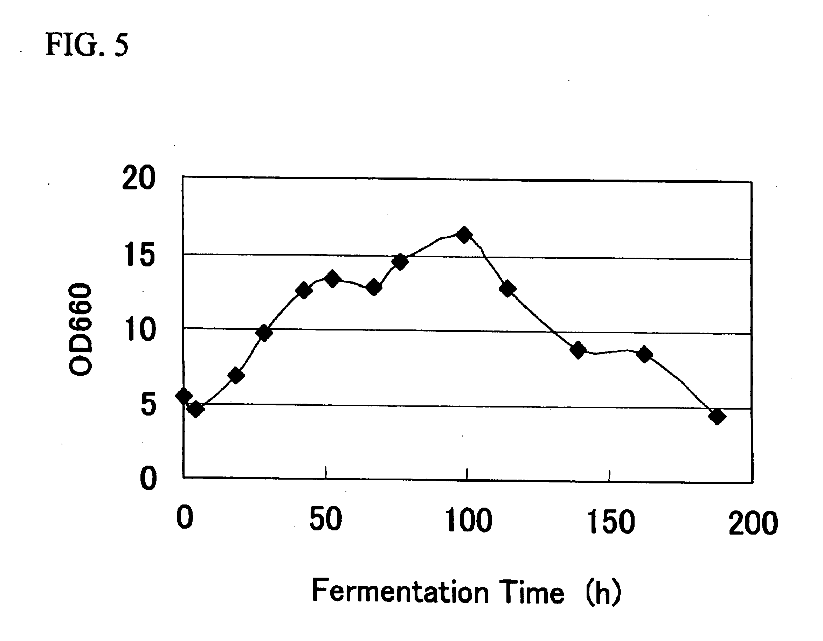

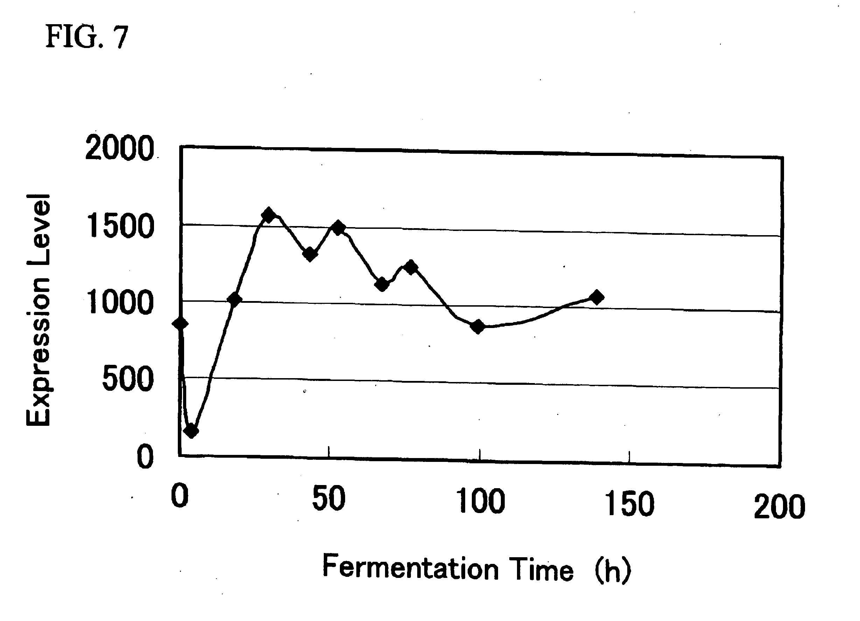

The fermentation liquor was sampled over time, and the time-course changes in amount of yeast cell growth (Fig. 5) and apparent extract concentration (Fig, 6) were observed. Simultaneously, yeast cells were sampled to prepare mRNA, and the prepared mRNA was labeled with biotin and was hybridized to a beer yeast DNA microarray. The signal was detected using GeneChip Operating system (GCOS; GeneChip Operating Software 1.0, manufactured by Affymetrix Co). Expression pattern of the non-ScGSY2 gene is shown in Figure 7. This result confirmed the expression of the non-ScGSY2 gene in the general beer fermentation.

### Example 8: Construction of non-ScGSY2 Highly Expressed Strain

The non-ScGSY2/pCR2.1-TOPO described in Example 6 was digested with the restriction enzymes SacI and NotI to prepare a DNA fragment containing the entire length of the protein-encoding region. This fragment was ligated to pYCGPYNot treated with the restriction enzymes SacI and NotI, thereby constructing the non-ScGSY2 high expression vector non-ScGSY2/pYCGPYNot.

Using the high expression vector prepared by the above method, an AJL4004 strain was transformed by the method described in Japanese Patent Application Laid-open No. H07-303475. The transformants were selected on a YPD plate medium (1% yeast extract, 2% polypeptone, 2% glucose and 2% agar) containing 300 mg/L of geneticin.

### Example 9: Evaluation of Drying-resistant Property of non-ScGSY2 Highly Expressed Strain

Drying-resistant properties of the parent strain (AJL4004 strain) and the non-ScGSY2 highly expressed strain obtained by the method described in Example 8 are evaluated by the same method as Example 4.

### Example 10: Evaluation of low-temperature Resistant Property of non-ScGSY2 Highly Expressed Strain

Low-temperature resistant property of the parent strain (AJL4004 strain) and the non-ScGSY2 highly expressed strain obtained by the method described in Example 8 are evaluated by the same method as Example 5.

### INDUSTRIAL APPLICABILITY

According to the present invention, yeast as defined in the claims can be stored stably for extended period of time, because drying-resistant property can be enhanced by the present invention. Accordingly, efficiency of brewing alcoholic beverages (such as beer), production of bread, or manufacturing useful materials such as industrial alcohol production or production of useful proteins, etc., can be improved by using the yeast of the present invention.

## Claims

1. A yeast comprising a vector containing the polynucleotide selected from:
(a) a polynucleotide comprising a polynucleotide consisting of the nucleotide sequence of SEQ ID NO: 1;
(b) a polynucleotide comprising a polynucleotide encoding a protein consisting of the amino acid sequence of SEQ ID NO: 2;
(c) a polynucleotide comprising a polynucleotide encoding a protein consisting of the amino acid sequence of SEQ ID NO: 2 in which 1 to 30 amino acids thereof are deleted, substituted, inserted and/or added, wherein said protein has a glycogen synthase activity and wherein amplifying expression of said protein in the yeast AJL4004 strain increases the drying-resistant property as compared to the AJL4004 strain;
(d) a polynucleotide comprising a polynucleotide encoding a protein having an amino acid sequence having 95% or higher identity with the amino acid sequence of SEQ ID NO: 2, wherein said protein has a glycogen synthase activity and wherein amplifying expression of said protein in the yeast AJL4004 strain increases the drying-resistant property as compared to the AJL4004 strain;
(e) a polynucleotide comprising a polynucleotide encoding a protein consisting of the amino acid sequence of SEQ ID NO: 2, or encoding the amino acid sequence of SEQ ID NO: 2 in which 1 to 7 amino acids thereof are deleted, substituted, inserted, and/or added, and wherein said protein has a glycogen synthase activity and wherein amplifying expression of said protein in the yeast AJL4004 strain increases the drying-resistant property as compared to the AJL4004 strain; and
(f) a polynucleotide comprising a polynucleotide encoding a protein having 99% or higher identity with the amino acid sequence of SEQ ID NO: 2, and having a glycogen synthase activity and wherein amplifying expression of said protein in the yeast AJL4004 strain increases the drying-resistant property as compared to the AJL4004 strain,
wherein drying-resistant property is increased by increasing an expression level of the protein encoded by the polynucleotide according to any one of (a) to (f).

2. A method for producing an alcoholic beverage by using the yeast according to Claim 1.

3. A method for producing an alcoholic beverage comprising: conducting fermentation using the yeast according to Claim 1.

## Patentansprüche

1. Hefe, umfassend einen Vektor, der das Polynucleotid enthält, das ausgewählt ist aus:
(a) einem Polynucleotid, umfassend ein Polynucleotid, das aus der Nucleotidsequenz der SEQ ID NO:1 besteht;
(b) einem Polynucleotid, umfassend ein Polynucleotid, das ein Protein codiert, das aus der Aminosäuresequenz der SEQ ID NO:2 besteht;
(c) einem Polynucleotid, umfassend ein Polynucleotid, das ein Protein codiert, das aus der Aminosäuresequenz der SEQ ID NO:2 besteht, in der 1 bis 30 Aminosäuren davon deletiert, substituiert, insertiert und/oder hinzugefügt sind, wobei das Protein eine Glycogensynthaseaktivität aufweist, und wobei das Amplifizieren der Expression des Proteins im Hefestamm AJL4004 die Eigenschaft der Trockenresistenz im Vergleich zum AJL4004-Stamm erhöht;
(d) einem Polynucleotid, umfassend ein Polynucleotid, das ein Protein codiert, das eine Aminosäuresequenz aufweist, die 95% oder mehr Identität mit der Aminosäuresequenz der SEQ ID NO:2 aufweist, wobei das Protein eine Glycogensynthaseaktivität aufweist, und wobei das Amplifizieren der Expression des Proteins im Hefestamm AJL4004 die Eigenschaft der Trockenresistenz im Vergleich zum AJL4004-Stamm erhöht;
(e) einem Polynucleotid, umfassend ein Polynucleotid, das ein Protein codiert, das aus der Aminosäuresequenz der SEQ ID NO:2 besteht, oder das die Aminosäuresequenz der SEQ ID NO:2 codiert, in der 1 bis 7 Aminosäuren davon deletiert, substituiert, insertiert und/oder hinzugefügt sind, und wobei das Protein eine Glycogensynthaseaktivität aufweist, und wobei das Amplifizieren der Expression des Proteins im Hefestamm AJL4004 die Eigenschaft der Trockenresistenz im Vergleich zum AJL4004-Stamm erhöht;
(f) einem Polynucleotid, umfassend ein Polynucleotid, das ein Protein codiert, das 99% oder mehr Identität mit der Aminosäuresequenz der SEQ ID NO:2 aufweist und das eine Glycogensynthaseaktivität hat, und wobei das Amplifizieren der Expression des Proteins im Hefestamm AJL4004 die Eigenschaft der Trockenresistenz im Vergleich zum AJL4004-Stamm erhöht,
wobei die Eigenschaft der Trockenresistenz erhöht wird durch Erhöhen eines Expressionsspiegels des Proteins, das von dem Polynucleotid nach einem beliebigen von (a) bis (f) codiert wird.

2. Verfahren zur Herstellung eines alkoholischen Getränks unter Verwendung der Hefe nach Anspruch 1.

3. Verfahren zur Herstellung eines alkoholischen Getränks, umfassend das Durchführen der Fermentation unter Verwendung der Hefe nach Anspruch 1.

## Revendications

1. Levure comprenant un vecteur contenant le polynucléotide choisi parmi :
(a) un polynucléotide comprenant un polynucléotide consistant en la séquence nucléotidique de SEQ ID NO : 1 ;
(b) un polynucléotide comprenant un polynucléotide codant pour une protéine consistant en la séquence d'acides aminés de SEQ ID NO : 2 ;
(c) un polynucléotide comprenant un polynucléotide codant pour une protéine consistant en la séquence d'acides aminés de SEQ ID NO : 2 dans laquelle 1 à 30 acides aminés de celle-ci sont délétés, substitués, insérés et/ou ajoutés, où ladite protéine possède une activité glycogène synthase et où l'amplification de l'expression de ladite protéine dans la souche AJL4004 de levure augmente la propriété de résistance au séchage comparativement à la souche AJL4004 ;
(d) un polynucléotide comprenant un polynucléotide codant pour une protéine ayant une séquence d'acides aminés présentant une identité de 95 % ou supérieure avec la séquence d'acides aminés de SEQ ID NO : 2, où ladite protéine possède une activité glycogène synthase et où l'amplification de l'expression de ladite protéine dans la souche AJL4004 de la levure augmente la propriété de résistance au séchage comparativement à la souche AJL4004 ;
(e) un polynucléotide comprenant un polynucléotide codant pour une protéine constituée de la séquence d'acides aminés de SEQ ID NO : 2, ou codant pour la séquence d'acides aminés de SEQ ID NO : 2 dans laquelle 1 à 7 acides aminés de celle-ci sont délétés, substitués, insérés et/ou ajoutés, et où ladite protéine possède une activité glycogène synthase et où l'amplification de l'expression de ladite protéine dans la souche AJL4004 de levure augmente la propriété de résistance au séchage comparativement à la souche AJL4004 ; et
(f) un polynucléotide comprenant un polynucléotide codant pour une protéine présentant une identité de 99 % ou supérieure avec la séquence d'acides aminés de SEQ ID NO : 2, et possédant une activité glycogène synthase et où l'amplification de l'expression de ladite protéine dans la souche AJL4004 de levure augmente la propriété de résistance au séchage comparativement à la souche AJL4004,
où la propriété de résistance au séchage est augmentée par l'augmentation d'un taux d'expression de la protéine codée par le polynucléotide selon l'un quelconque de (a) à (f).

2. Procédé de production d'une boisson alcoolisée en utilisant la levure selon la revendication 1.

3. Procédé de production d'une boisson alcoolisée comprenant : la conduite d'une fermentation en utilisant la levure selon la revendication 1.


### SEQUENCE LISTING

<110> Suntory Limited
<120> Gene encoding glycogen synthase and use thereof
<130> PCT06-0144
<150> JP2006-54195
   <151> 2006-02-28
<160> 8
<170> Patent In version 3.3
<210> 1
   <211> 2130
   <212> DNA
   <213> Saccharomyces sp.
<400> 1
   atggctcgtg acttacaaaa ccatcttcta ttcgaagtag caacggaagt cgccaacagg      60
   gttggtggca tttactctgt cttgaaatcc aaggccccag ttaccgtggc tcagtacgga     120
   gacaactata cgctgatcgg gcccttgaac aagtccacgt atgaaggtga ggtcgaaaaa     180
   ctcgactggg aggacgaaga ggttttccgc gaggacctat tgcccattca aaggacgttg     240
   atgtcgatgc gcgaaaaggg tgtgaatttc gtctacggtc gttggttggt ggagggagca     300
   ccacgtgtca ttttgtttga cctggactct gtaagacacc tcctgggcga atggaaggct     360
   gatctatggt cccttgtggg aatcccgtct cccgagcacg accacgaaac aaacgacgct     420
   atcttatttg gctacgttgt tgtgtggttt ttgggagagg tatcgaaact ggacaacaaa     480
   catgcaatca tcggccactt ccacgaatgg ttggccggtg tggccctacc attgtgtcgg     540
   aagaaaagaa ttgacgtcgt gacaattttt accacgcacg ctaccttatt gggtagatac     600
   ttgtgtgccg cgggtgatgt ggacttttat aatcaattac agcttttcga tgtggaccag     660
   gaagctggca aaaggggaat ttatcacagg tactgcatcg agcgtgccgc cgctcacact     720
   gcggacgttt tcaccactgt gtcacagatc actgcattcg aagcggagca tttgttgaag     780
   aggaagcctg acggtatctt gcccaatggg ttgaatgtcg tcaagttcca agccgttcat     840
   gagttccaaa acctgcatgc actgaaaaaa gagaagatca atgaattcgt tagaggtcat     900
   ttccatggct gtttcgattt cgatctagac aacaccgttt acttctttat cgctggtaga     960
   tatgaataca aaaacaaagg tgccgacatg ttcatcgagt ccttggctcg tcttaattac    1020
   agattgaagg tttccgggtc taagaaaact gtggtggcat ttttgattat gcctgctaag    1080
   accaattctt tcactgtgga agcactaaag agccaagcta ttgtgaaatc cttggaaaac    1140
   acagtcgacg agatcactgc atccattggc aaaagaatat tcgagcatac aatgagatat 1200
   cctcataacg gtctagagac agaattgccc actaatctag acgaactatt gaaaagttct 1260
   gaaaaagtgt tattgaaaaa aagagttttg gcattgagaa gacctcacgg cgaattacct 1320
   ccagtggtca cacataatat gtgtgatgat gctaacgatc caatcctgaa tcaaatcaga 1380
   cacgtcagat tattcaacga tccaagcgat cgtgtcaaag tcatattcca ccctgagttt 1440
   ttgaacgcaa ataatcctat tctcggactg gattacgatg aattcgttcg tggttgccat 1500
   ttgggtgtct tcccatccta ctatgaacca tgggggtaca cgccggcaga atgtaccgtt 1560
   atgggggttc cctcaatcac aacaaacgtt tccgggtttg gtgcctatat ggaggacttg 1620
   atcgaaactg atcaggctaa ggactacggt atttatatcg tggaccgtcg tttcaaagcc 1680
   cccgatgaat ctgtggagca attagccgac tacatggaag aatttgttaa caagactaga 1740
   agacaaagaa ttaaccaaag aaacagaacc gagagactat ctgatctgtt agattggaag 1800
   agaatggggt tggaatacgt caaggctaga caattgggtc ttcgtagagc ttacccagac 1860
   cagttcaagg aattgatagg tgaagatatt agcgacgcca atatgaatac ccttgccggt 1920
   ggtaagaagt ttaagattgc cagacctcta agtgtacctg gatctcccaa ggtaagatcc 1980
   aacagtaccg tttacatgac tccgggcgac ctgggaaccc tccaagacgc caacaatgcg 2040
   gatgattact ttaatctaag catgaatgga ggcgttgcag gtgatgatga cgacgacgat 2100
   gacacttctg catactatga agataactga 2130
<210> 2
   <211> 709
   <212> PRT
   <213> Saccharomyces sp.
<400> 2
   Met Ala Arg Asp Leu Gln Asn His Leu Leu Phe Glu Val Ala Thr Glu
   1 5 10 15
   Val Ala Asn Arg Val Gly Gly Ile Tyr Ser Val Leu Lys Ser Lys Ala
   20 25 30
   Pro Val Thr Val Ala Gln Tyr Gly Asp Asn Tyr Thr Leu Ile Gly Pro
   35 40 45
   Leu Asn Lys Ser Thr Tyr Glu Gly Glu Val Glu Lys Leu Asp Trp Glu
   50 55 60
   Asp Glu Glu Val Phe Arg Glu Asp Leu Leu Pro Ile Gln Arg Thr Leu
   65 70 75 80
   Met Ser Met Arg Glu Lys Gly Val Asn Phe Val Tyr Gly Arg Trp Leu
   85 90 95
   Val Glu Gly Ala Pro Arg Val Ile Leu Phe Asp Leu Asp Ser Val Arg
   100 105 110
   His Leu Leu Gly Glu Trp Lys Ala Asp Leu Trp Ser Leu Val Gly Ile
   115 120 125
   Pro Ser Pro Glu His Asp His Glu Thr Asn Asp Ala Ile Leu Phe Gly
   130 135 140
   Tyr Val Val Val Trp Phe Leu Gly Glu Val Ser Lys Leu Asp Asn Lys
   145 150 155 160
   His Ala Ile Ile Gly His Phe His Glu Trp Leu Ala Gly Val Ala Leu
   165 170 175
   Pro Leu Cys Arg Lys Lys Arg Ile Asp Val Val Thr Ile Phe Thr Thr
   180 185 190
   His Ala Thr Leu Leu Gly Arg Tyr Leu Cys Ala Ala Gly Asp Val Asp
   195 200 205
   Phe Tyr Asn Gln Leu Gln Leu Phe Asp Val Asp Gln Glu Ala Gly Lys
   210 215 220
   Arg Gly Ile Tyr His Arg Tyr Cys Ile Glu Arg Ala Ala Ala His Thr
   225 230 235 240
   Ala Asp Val Phe Thr Thr Val Ser Gln Ile Thr Ala Phe Glu Ala Glu
   245 250 255
   His Leu Leu Lys Arg Lys Pro Asp Gly Ile Leu Pro Asn Gly Leu Asn
   260 265 270
   Val Val Lys Phe Gln Ala Val His Glu Phe Gln Asn Leu His Ala Leu
   275 280 285
   Lys Lys Glu Lys Ile Asn Glu Phe Val Arg Gly His Phe His Gly Cys
   290 295 300
   Phe Asp Phe Asp Leu Asp Asn Thr Val Tyr Phe Phe Ile Ala Gly Arg
   305 310 315 320
   Tyr Glu Tyr Lys Asn Lys Gly Ala Asp Met Phe Ile Glu Ser Leu Ala
   325 330 335
   Arg Leu Asn Tyr Arg Leu Lys Val Ser Gly Ser Lys Lys Thr Val Val
   340 345 350
   Ala Phe Leu Ile Met Pro Ala Lys Thr Asn Ser Phe Thr Val Glu Ala
   355 360 365
   Leu Lys Ser Gln Ala Ile Val Lys Ser Leu Glu Asn Thr Val Asp Glu
   370 375 380
   Ile Thr Ala Ser Ile Gly Lys Arg Ile Phe Glu His Thr Met Arg Tyr
   385 390 395 400
   Pro His Asn Gly Leu Glu Thr Glu Leu Pro Thr Asn Leu Asp Glu Leu
   405 410 415
   Leu Lys Ser Ser Glu Lys Val Leu Leu Lys Lys Arg Val Leu Ala Leu
   420 425 430
   Arg Arg Pro His Gly Glu Leu Pro Pro Val Val Thr His Asn Met Cys
   435 440 445
   Asp Asp Ala Asn Asp Pro Ile Leu Asn Gln Ile Arg His Val Arg Leu
   450 455 460
   Phe Asn Asp Pro Ser Asp Arg Val Lys Val Ile Phe His Pro Glu Phe
   465 470 475 480
   Leu Asn Ala Asn Asn Pro Ile Leu Gly Leu Asp Tyr Asp Glu Phe Val
   485 490 495
   Arg Gly Cys His Leu Gly Val Phe Pro Ser Tyr Tyr Glu Pro Trp Gly
   500 505 510
   Tyr Thr Pro Ala Glu Cys Thr Val Met Gly Val Pro Ser Ile Thr Thr
   515 520 525
   Asn Val Ser Gly Phe Gly Ala Tyr Met Glu Asp Leu Ile Glu Thr Asp
   530 535 540
   Gln Ala Lys Asp Tyr Gly Ile Tyr Ile Val Asp Arg Arg Phe Lys Ala
   545 550 555 560
   Pro Asp Glu Ser Val Glu Gln Leu Ala Asp Tyr Met Glu Glu Phe Val
   565 570 575
   Asn Lys Thr Arg Arg Gln Arg Ile Asn Gln Arg Asn Arg Thr Glu Arg
   580 585 590
   Leu Ser Asp Leu Leu Asp Trp Lys Arg Met Gly Leu Glu Tyr Val Lys
   595 600 605
   Ala Arg Gln Leu Gly Leu Arg Arg Ala Tyr Pro Asp Gln Phe Lys Glu
   610 615 620
   Leu Ile Gly Glu Asp Ile Ser Asp Ala Asn Met Asn Thr Leu Ala Gly
   625 630 635 640
   Gly Lys Lys Phe Lys Ile Ala Arg Pro Leu Ser Val Pro Gly Ser Pro
   645 650 655
   Lys Val Arg Ser Asn Ser Thr Val Tyr Met Thr Pro Gly Asp Leu Gly
   660 665 670
   Thr Leu Gln Asp Ala Asn Asn Ala Asp Asp Tyr Phe Asn Leu Ser Met
   675 680 685
   Asn Gly Gly Val Ala Gly Asp Asp Asp Asp Asp Asp Asp Thr Ser Ala
   690 695 700
   Tyr Tyr Glu Asp Asn
   705
<210> 3
   <211> 2118
   <212> DNA
   <213> Saccharomyces sp.
<400> 3
   atgtcccgtg atctacagaa tcatttattg ttcgagaccg caactgaggt tgctaatagg 60
   gtcggtggta tttactccgt gctaaaatca aaggccccca ttactgtcgc ccagtacaag 120
   gacaattacc atttaatagg gcccttgaat aaggccactt accaaaatga ggttgatata 180
   ctggattgga agaaacctga agccttttcc gaggatttga agcctattca acatgctttg 240
   caaacgatgg aatctagagg tgttcatttt gtttatggga gatggttaat tgaaggcgct 300
   ccaagagtga ttctttttga tttggactct gtgagaggct tttcggacga gtggaagggc 360
   gatttatggt cattggtagg aattccctct cccgagaatg atttcgagac gaatgatgcc 420
   atcttattgg gctacacggt ggcttggttc ctaggtgaat tggctcactt ggattctcaa 480
   catgccattg ttgcacattt ccatgaatgg ttggccggtg tcgcgatacc cttgtgccgg 540
   aaaagacgta ttgatgtagt tacgattttc actacccacg ctactttatt gggaaggtat 600
   ctatgtgcct ctggtagttt cgatttttac aattgcttag aaaccgttga cgtcgatcat 660
   gaagctggaa aatttggtat ttaccatcgt tactgtatag aaagagccgc tgctcattcc 720
   gccgacgttt tcaccacagt gtcacaaatt accgcttttg aagcagaaca tcttttgaaa 780
   agaaaaccag atgggatctt gcctaacggg ctgaacgtaa tcaaattcca agcattccat 840
   gagttccaga atttgcatgc tttgaaaaag gagaaaatca acgattttgt tagaggccac 900
   tttcacggct gctttgattt cgacttggac aataccttgt acttctttat cgctggtaga 960
   tatgagtata aaaataaggg tgctgacatg tttattgaag ctctagctcg tttaaactat 1020
   agactgaaag tatctggctc taagaaaacc gttgtggcgt ttattgtcat gcctgccaag 1080
   aacaattcat ttacagtgga ggcattaaaa ggccaagcag aagtgaaagc cttagaaaac 1140
   actgtagatg aagtgaccgc ctcaattggt aagcggatat ttgaccacgc catcaggtac 1200
   ccacacaatg ggttgacttc cgaattgcca acagatttag gcgaattgct gaagagttct 1260
   gataaagtca tgttaaaaag acgcattcta gctttgagaa gacctgcggg agaattacct 1320
   ccaatagtga cacacaacat gattgacgat gctaatgatt tgattttgaa taaaatcaga 1380
   catgttcaat tattcaacag tccaagtgac cgtgtcaaaa tgattttcca tccagaattt 1440
   ttgaacgcaa ataatccaat tcttggttta gactatgacg aattcgttcg tggttgccat 1500
   ttgggtgtct tcccttcata ctatgaacct tggggataca ccccagcaga atgtaccgta 1560
   atgggtgttc cttctatcac aacgaacgtc tctgggtttg gggcttatat ggaagactta 1620
   atcgagacca accaagctaa agattatggt atttatattg tagatcgtcg ctttaaaggg 1680
   ccagatgaat ctgtggaaca actggttgat tatatggaag agttcgtgaa aaagacaaga 1740
   aggcaaagaa taaatcaaag aaacagaacc gaaagactat ctgacttgtt ggactggaaa 1800
   agaatggggc tcgaatacgt aaaggcaagg caattggcat taaggagagc ataccctgat 1860
   caattcaaag aattggttgg ggaagaactc aatgattcta acatggactc tttagctggg 1920
   ggtaagaaac taaaagtcgc aaggccgcta agtgtaccag gctcgccaag agatttgaga 1980
   tcaaatagta cagtttacat gactcctggt gatttaggga ccttacaaga ggctaataat 2040
   gcggacgatt atttctcttt gggcgtgaat cctgcagccg atgatgatga cgatggccca 2100
   tatgccgatg atagttaa 2118
<210> 4
   <211> 705
   <212> PRT
   <213> Saccharomyces sp.
<400> 4
   Met Ser Arg Asp Leu Gln Asn His Leu Leu Phe Glu Thr Ala Thr Glu
   1               5                   10                  15
   Val Ala Asn Arg Val Gly Gly Ile Tyr Ser Val Leu Lys Ser Lys Ala
               20                  25                  30
   Pro Ile Thr Val Ala Gln Tyr Lys Asp Asn Tyr His Leu Ile Gly Pro
           35                  40                  45
   Leu Asn Lys Ala Thr Tyr Gln Asn Glu Val Asp Ile Leu Asp Trp Lys
       50                  55                  60
   Lys Pro Glu Ala Phe Ser Glu Asp Leu Lys Pro Ile Gln His Ala Leu
   65                  70                  75                  80
   Gln Thr Met Glu Ser Arg Gly Val His Phe Val Tyr Gly Arg Trp Leu
                   85                  90                  95
   Ile Glu Gly Ala Pro Arg Val Ile Leu Phe Asp Leu Asp Ser Val Arg
               100                 105                 110
   Gly Phe Ser Asp Glu Trp Lys Gly Asp Leu Trp Ser Leu Val Gly Ile
           115                 120                 125
   Pro Ser Pro Glu Asn Asp Phe Glu Thr Asn Asp Ala Ile Leu Leu Gly
       130                 135                 140
   Tyr Thr Val Ala Trp Phe Leu Gly Glu Leu Ala His Leu Asp Ser Gln
   145                 150                 155                 160
   His Ala Ile Val Ala His Phe His Glu Trp Leu Ala Gly Val Ala Ile
                   165                 170                 175
   Pro Leu Cys Arg Lys Arg Arg Ile Asp Val Val Thr Ile Phe Thr Thr
               180                 185                 190
   His Ala Thr Leu Leu Gly Arg Tyr Leu Cys Ala Ser Gly Ser Phe Asp
           195                 200                 205
   Phe Tyr Asn Cys Leu Glu Thr Val Asp Val Asp His Glu Ala Gly Lys
       210                 215                 220
   Phe Gly Ile Tyr His Arg Tyr Cys Ile Glu Arg Ala Ala Ala His Ser
   225 230 235 240
   Ala Asp Val Phe Thr Thr Val Ser Gln Ile Thr Ala Phe Glu Ala Glu
   245 250 255
   His Leu Leu Lys Arg Lys Pro Asp Gly Ile Leu Pro Asn Gly Leu Asn
   260 265 270
   Val Ile Lys Phe Gln Ala Phe His Glu Phe Gln Asn Leu His Ala Leu
   275 280 285
   Lys Lys Glu Lys Ile Asn Asp Phe Val Arg Gly His Phe His Gly Cys
   290 295 300
   Phe Asp Phe Asp Leu Asp Asn Thr Leu Tyr Phe Phe Ile Ala Gly Arg
   305 310 315 320
   Tyr Glu Tyr Lys Asn Lys Gly Ala Asp Met Phe Ile Glu Ala Leu Ala
   325 330 335
   Arg Leu Asn Tyr Arg Leu Lys Val Ser Gly Ser Lys Lys Thr Val Val
   340 345 350
   Ala Phe Ile Val Met Pro Ala Lys Asn Asn Ser Phe Thr Val Glu Ala
   355 360 365
   Leu Lys Gly Gln Ala Glu Val Lys Ala Leu Glu Asn Thr Val Asp Glu
   370 375 380
   Val Thr Ala Ser Ile Gly Lys Arg Ile Phe Asp His Ala Ile Arg Tyr
   385 390 395 400
   Pro His Asn Gly Leu Thr Ser Glu Leu Pro Thr Asp Leu Gly Glu Leu
   405 410 415
   Leu Lys Ser Ser Asp Lys Val Met Leu Lys Arg Arg Ile Leu Ala Leu
   420 425 430
   Arg Arg Pro Ala Gly Glu Leu Pro Pro Ile Val Thr His Asn Met Ile
   435 440 445
   Asp Asp Ala Asn Asp Leu Ile Leu Asn Lys Ile Arg His Val Gln Leu
   450 455 460
   Phe Asn Ser Pro Ser Asp Arg Val Lys Met Ile Phe His Pro Glu Phe
   465 470 475 480
   Leu Asn Ala Asn Asn Pro Ile Leu Gly Leu Asp Tyr Asp Glu Phe Val
   485 490 495
   Arg Gly Cys His Leu Gly Val Phe Pro Ser Tyr Tyr Glu Pro Trp Gly
   500 505 510
   Tyr Thr Pro Ala Glu Cys Thr Val Met Gly Val Pro Ser Ile Thr Thr
   515 520 525
   Asn Val Ser Gly Phe Gly Ala Tyr Met Glu Asp Leu Ile Glu Thr Asn
   530 535 540
   Gln Ala Lys Asp Tyr Gly Ile Tyr Ile Val Asp Arg Arg Phe Lys Gly
   545 550 555 560
   Pro Asp Glu Ser Val Glu Gln Leu Val Asp Tyr Met Glu Glu Phe Val
   565 570 575
   Lys Lys Thr Arg Arg Gln Arg Ile Asn Gln Arg Asn Arg Thr Glu Arg
   580 585 590
   Leu Ser Asp Leu Leu Asp Trp Lys Arg Met Gly Leu Glu Tyr Val Lys
   595 600 605
   Ala Arg Gln Leu Ala Leu Arg Arg Ala Tyr Pro Asp Gln Phe Lys Glu
   610 615 620
   Leu Val Gly Glu Glu Leu Asn Asp Ser Asn Met Asp Ser Leu Ala Gly
   625 630 635 640
   Gly Lys Lys Leu Lys Val Ala Arg Pro Leu Ser Val Pro Gly Ser Pro
   645 650 655
   Arg Asp Leu Arg Ser Asn Ser Thr Val Tyr Met Thr Pro Gly Asp Leu
   660 665 670
   Gly Thr Leu Gln Glu Ala Asn Asn Ala Asp Asp Tyr Phe Ser Leu Gly
   675 680 685
   Val Asn Pro Ala Ala Asp Asp Asp Asp Asp Gly Pro Tyr Ala Asp Asp
   690 695 700
   Ser
   705
<210> 5
   <211> 40
   <212> DNA
   <213> Artificial
<220>
   <223> Primer
<400> 5
   gagctcatag cggccatggc tcgtgactta caaaaccatc 40
<210> 6
   <211> 42
   <212> DNA
   <213> Artificial
<220>
   <223> Primer
<400> 6
   ggatcctatg cggccgcgta cttctacaaa ggttatataa ac 42
<210> 7
   <211> 40
   <212> DNA
   <213> Artificial
<220>
   <223> Primer
<400> 7
   gagctcatag cggccatgtc ccgtgatcta cagaatcatt 40
<210> 8
   <211> 42
   <212> DNA
   <213> Artificial
<220>
   <223> Primer
<400> 8
   ggatcctatg cggccgctat atatacgaaa aaggcatggc aa 42